# EUROPEAN PATENT APPLICATION

(11) **EP 1 903 052 A2**
(43) Date of publication of application: **26.03.2008**
(21) Application number: 06291229.0
(22) Date of filing: 28.07.2006
(51) Int. Cl.: C07K 5/068, A61K 31/427

(54) **APJ receptor ligands and uses thereof**

(71) Applicant: Faust Pharmaceuticals SA, 67400 Illkirch Graffenstaden (FR); INSTITUT NATIONAL DE LA SANTE ET DE LA RECHERCHE MEDICALE (INSERM), 75654 Paris Cédex 13 (FR)
(72) Inventor: Llorens-Cortes, Catherine, 91440 Bures-sur-Yvette (FR); Guillier, Fabrice, F-67118 Geispolsheim (FR); Franchet, Christel, F-67120 Ernolsheim sur Bruche (FR)
(74) Representative: Peaucelle, Chantal

(57) **Abstract**

The present invention provides novel ligands of formula (I) for the G-protein-coupled orphan receptor APJ, and uses thereof for treating and/or preventing diseases, conditions and/or disorders mediated by APJ receptors. The invention further relates to assays for the identification of agents able to modulate APJ ligand binding, signalling and biological activities.

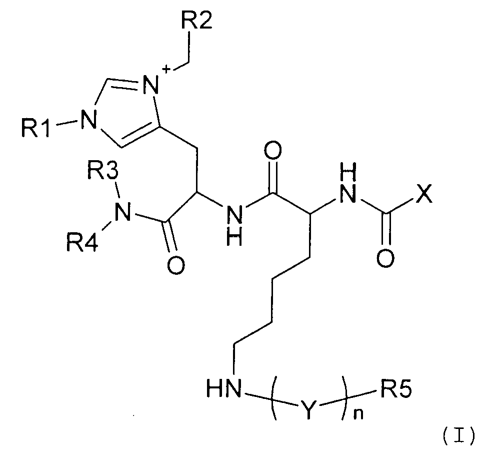

## Description

The present invention provides novel ligands for the G-protein-coupled orphan receptor APJ, and uses thereof for treating and/or preventing diseases, conditions and/or disorders mediated by APJ receptors. The invention further relates to assays for the identification of agents able to modulate APJ ligand binding, signalling and biological activities.

The orphan receptor APJ (putative receptor protein related to angiotensin II type 1 receptor or AT1) was first identified in a human gene (O'Dowd et al., Gene, 1993). The human APJ receptor is a G-protein coupled receptor with seven transmembrane domains, constituted of 380 amino acids. However, this APJ receptor displays no specific binding for angiotensin II or angiotensin III. Moreover, stimulation of this receptor by angiotensin II or angiotensin III does not modify cAMP production (De Mota et al., Neuroendocrinology, 2000).

In the search for an endogenous ligand of the orphan receptor APJ, a peptide called apelin (APJ endogenous ligand) was first isolated from bovine stomach extracts and the corresponding human protein was deduced from this discovery (Tatemoto et al., Biochemical and Biophysical Research Communications, 1998). The apelin polypeptide is initially produced as a 77 amino acid protein (called proapelin) that is cleaved to produce cleavage products of 36 amino acids, 17 amino acids, and 13 amino acids, each of them having a high affinity (in the nM range) for the APJ receptor. The peptide consisting of the C-terminal 13 amino acids of the apelin polypeptide is necessary and sufficient for the ability of an apelin polypeptide to interact with APJ.

Ligands for APJ receptor, which is expressed in the central nervous system, cardiovascular system (i.e. heart, kidney and vessels), reproductive system, immune system, digestive system and so forth, are expected to be useful as drugs. Apelin and APJ receptors are both widely distributed in the brain but are particularly highly expressed in the supraoptic (SON) and paraventricular (PVN) hypothalamic nuclei. Dual labelling studies demonstrate that within these two nuclei, apelin and its receptor are colocalized with vasopressin (AVP) in a subset of magnocellular neurons. In lactating rats, characterized by increases in both synthesis and release of AVP, central injection of apelin inhibits the phasic electrical activity of AVP neurons, decreases systemic AVP release inducing aqueous diuresis. Taken together, these data suggest that apelin is a natural inhibitor of the antidiuretic effect of AVP (De Mota et al., PNAS, 2004, Reaux et al., Endocrinology, 2004). Moreover apelin systemically administered reduces arterial blood pressure (Lee et al., J. Neurochem., 2000; Reaux et al., J. Neurochem., 2001; Tatemoto et al., Regulatory Peptides, 2001) increases cardiac contractility and reduces cardiac loading (Ashley et al., Cardiovascular Research, 2005).

Moreover, studies showed that APJ receptor acts as a coreceptor for the entry of several HIV-1 and SIV strains (Edinger et al., Journal of Virology, 1998 and Choe et al., Journal of Virology, 1998). Therefore, ligands to APJ receptor could be useful as drugs for the treatment and/or prophylaxis of diseases and conditions mediated by APJ signalling.

Using a FRET (Fluorescence Resonance Energy Transfer) based screening method, the inventors have surprisingly discovered new non-peptidic ligands able to bind the APJ receptor.

### Description:

The present invention provides compounds of formula (I) that act as APJ receptor ligands: 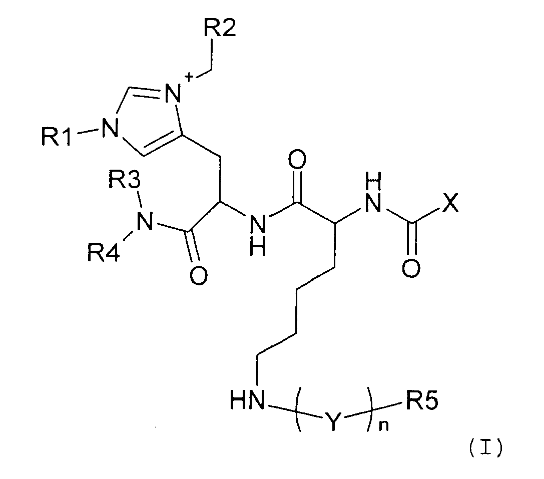 wherein:
**R1** is an aryl, alkylaryl, heteroaryl or alkylheteroaryl group;
**R2** is a hydrogen atom or an aryl group;
**R3** and **R4** represent a hydrogen atom or a heterocycloalkyl group providing that **R3** and **R4** cannot represent simultaneously a hydrogen atom and that **R3** and **R4** can both be part of a heterocycloalkyl group;
**R5** represents a group chosen is the following list: 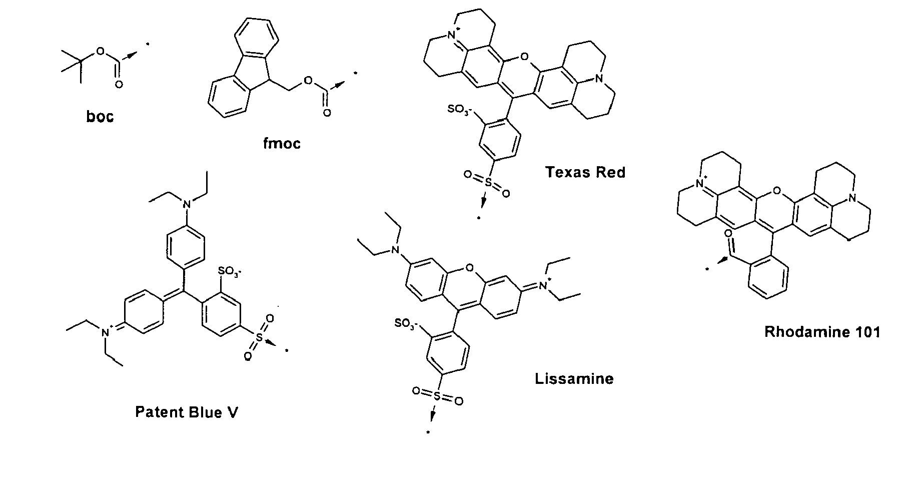
**n** is an integer from 0 to 1
**Y** represents -CO-(NH)ₙ,-A-NH- group with:
   **n'** is an integer from 0 to 1;
   **A** is a group chosen is the following list:
      -(CH₂)ₙ"-
      -[(CH₂)₂-O]_{n'"}-(CH₂)₂-
      -(CH₂)ₘ-NH-CO-(CH₂)_{m'}-
      -(CH₂)ₘ-NH-CO-(CH₂)_{m'}-NH-CO-(CH₂)_{m"}-
      -(CH₂)ₘ-CO-NH-(CH₂)_{m'}-
      -(CH₂)ₘ-CO-NH-(CH₂)_{m'}-CO-NH-(CH₂)_{m"}-
      with n" representing an integer ranging from 1 to 20;
      with n"' representing an integer ranging from 1 to 10;
      with m, m' and m" representing independently from the other an integer ranging from 1 to 15;
**X** represents a group chosen in the following list: 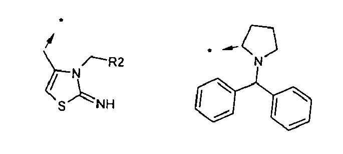 as well as a pharmaceutically acceptable salt thereof, a prodrug of the compound or the salt, or a solvate or hydrate of the compound, the salt or the prodrug.

As used herein, the term **"alkyl"** includes straight or branched chain saturated hydrocarbon residues with 1-8 carbon atoms, such as methyl, ethyl, n-propyl, isopropyl, n-butyl, i-butyl, s-butyl and t-butyl. These alkyls can be further substituted with one or several groups such as COOH, heterocycloalkyl, CF₃, OH, O-alkyl or N-(lower alkyl)(lower alkyl) .

As used herein, the term **"lower alkyl"** includes straight or branched chain saturated hydrocarbon residues with 1-4 carbon atoms, such as methyl, ethyl, n-propyl, isopropyl, n-butyl, i-butyl, s-butyl or t-butyl.

The term **"aryl"** refers to a 6-10 atoms aromatic hydrocarbon ring or fused aromatic hydrocarbon ring system containing at least one unsaturated aromatic ring. Examples of the term "aryl" are phenyl, naphthyl and 1,2,3,4-tetrahydronaphthyl. These aryls can be further substituted with one or several groups such as halogen, CN, CF₃, OCF₃, lower alkyl, COOH, O-(lower alkyl), S-(lower alkyl), N-(lower alkyl)(lower alkyl) or heterocycloalkyl.

The term **"heteroaryl"** means a 5-10 atoms aromatic ring or fused aromatic rings containing one or more O, S, or N atoms. Examples of heteroaryls include pyridinyl, quinolinyl, isoquinolinyl, pyridazinyl, pyrimidinyl, pyrazinyl, furyl, benzofuryl, thienyl, benzothienyl, pyrrolyl, indolyl, pyrazolyl, indazolyl, oxazolyl, benzoxazolyl, thiazolyl, benzothiazolyl, imidazolyl, benzimidazolyl, and tetrazolyl. These heteroaryls can be further substituted with one or several groups such as halogen, CN, CF₃, OCF₃, lower alkyl, COOH, O-(lower alkyl), S-(lower alkyl), N-(lower alkyl) (lower alkyl) or heterocycloalkyl.

The term **"heterocycloalkyl"** means a 4-6 atoms ring containing one or more O, S, or N atoms. Examples of heterocycloalkyls include azetidinyl, pyrrolidinyl, tetrahydrofuranyl, imidazolinyl, pyrolidin-2-one, morpholinyl, thiomorpholinyl, piperidinyl, piperidin-2-one, piperazinyl, N-alkyl-piperazinyl, N-alkylaryl-piperazinyl.

The term **"alkylaryl"** means an alkyl-aryl-group or radical wherein alkyl and aryl have the meanings as defined above. Illustrative examples of an alkylaryl group or radical include benzyl, 2-phenylethyl, 3-phenylpropyl, 4-phenylbutyl, 3-methyl-3-phenylpropyl, 1-naphthylmethyl, 1-naphthylethyl.

The term **"alkylheteroaryl"** means an alkyl-heteroaryl-group or radical wherein alkyl and heteroaryl have the meanings as defined above.

The term **"halogen"** refers to bromine, chlorine, fluorine, or iodine.

In a particular embodiment, the compound of the invention is of formula (II): 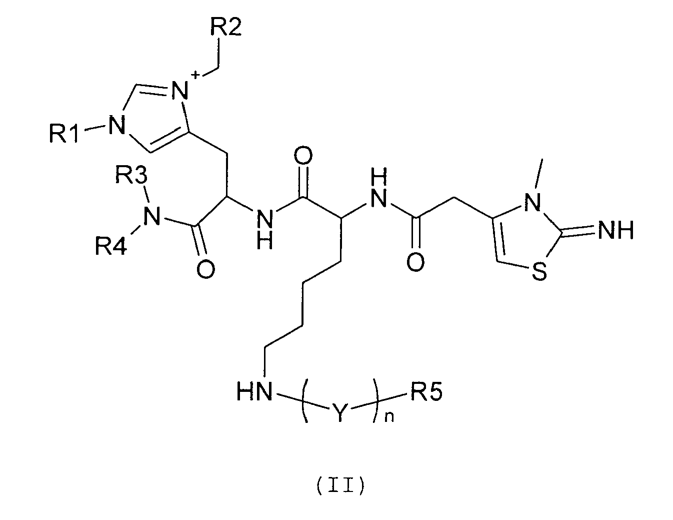 with:
**R1** to **R5**, **n** and **Y** being as defined above.

In a further embodiment, the compound of the invention is of formula (III): 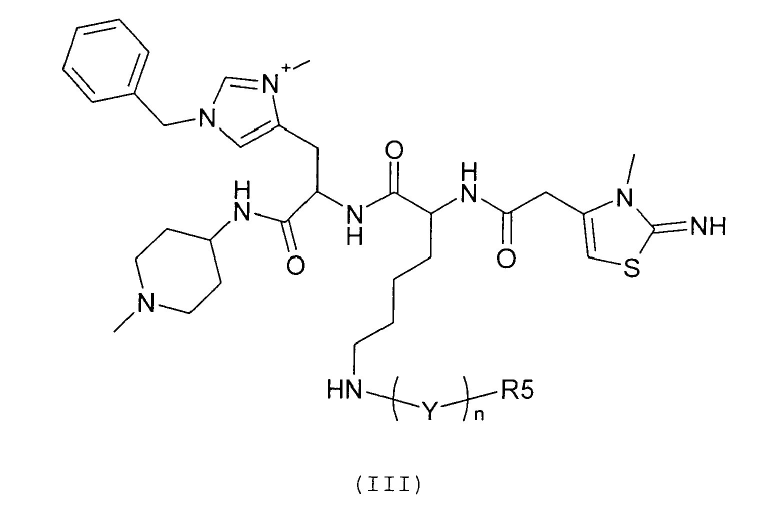 with:
**R5**, **n** and **Y** being as defined above.

The compounds of the invention are associated with the modulation of central nervous system function (vasopressin neuron activity and systemic vasopressin release , drinking behavior, food intake), cardiovascular function (blood pressure, myocardium contractibility), immune function, gastrointestinal function, metabolic function, reproductive function, etc..., and therefore, can be used as a therapeutic and/or prophylactic agent for a variety of diseases.

The present invention is also encompassing a method for treating and/or preventing a disease, condition or disorder mediated by the APJ receptor in mammals, such method involving the step of administering to a mammal in need thereof a therapeutically effective amount of a compound of the present invention or a pharmaceutical composition thereof.
Diseases, conditions and/or disorders which could be treated or prevented by the administration of a compound of the invention are for example:
- Innapropriate vasopressin secretions (SIADH) including pathologies like neurogenic diabetes mellitus (e.g. diabetic complications such as diabetic nephropathy, diabetic neuropathy, diabetic retinopathy, etc.), lung cancer, septic choc, thirst troubles;
- Cardiovascular diseases: Heart failure, diseases of kidney (e.g. renal failure, nephritis, etc.) hypertension, cirrhosis, arteriosclerosis, pulmonary emphysema, pulmonary oedema;
- Metabolic diseases: Obesity, anorexia, hyperphagia, polyphagia, hypercholesterolemia, hyperglyceridemia, hyperlipemia;
- Various types of dementia such as senile dementia, cerebrovascular dementia, dementia due to genealogical denaturation degenerative diseases (e.g. Alzheimer's disease, Parkinson's disease, Pick's disease, Huntington's disease, etc.), dementia resulting from infectious diseases (e.g. delayed virus infections such as Creutzfeldt-Jakob disease), dementia associated with endocrine diseases, metabolic diseases, or poisoning (e.g. hypothyroidism, vitamin B12 deficiency, alcoholism, poisoning caused by various drugs, metals, or organic compounds), dementia caused by tumors (e.g. brain tumor), and dementia due to traumatic diseases (e.g. chronic subdural hematoma), depression, hyperactive child syndrome (microencephalopathy), disturbance of consciousness, anxiety disorder, schizophrenia, phobia;
- Growth hormone secretory disorder (e.g. gigantism, acromegaly, etc.), hyperprolactinemia. galactorrhea.
- cancer (e.g. mammary cancer, lymphocytic leukemia, bladder cancer, ovary cancer, carcinoma of prostate, etc.);
- pancreatitis, Turner's syndrome, neurosis, rheumatoid arthritis, spinal cord injury, transient brain ischemia, amyotrophic lateral sclerosis, spinocerebellar degeneration, bone fracture, wounds, atopic dermatitis, osteoporosis, asthma, epilepsy, sterility.

It can further be used as a postoperative nutritional status improving agent or as an inotropic agent, vasodilatator or an aqueous diuretic. In addition, it can be used as drug for treating or preventing HIV infection or AIDS (acquired immune deficiency syndrome) or the like.

The compounds of the invention can be obtained using well known procedures. As an example, the following one describes the synthesis scheme of the compound of formula (III) coupled to lissamine (fluorescent group):

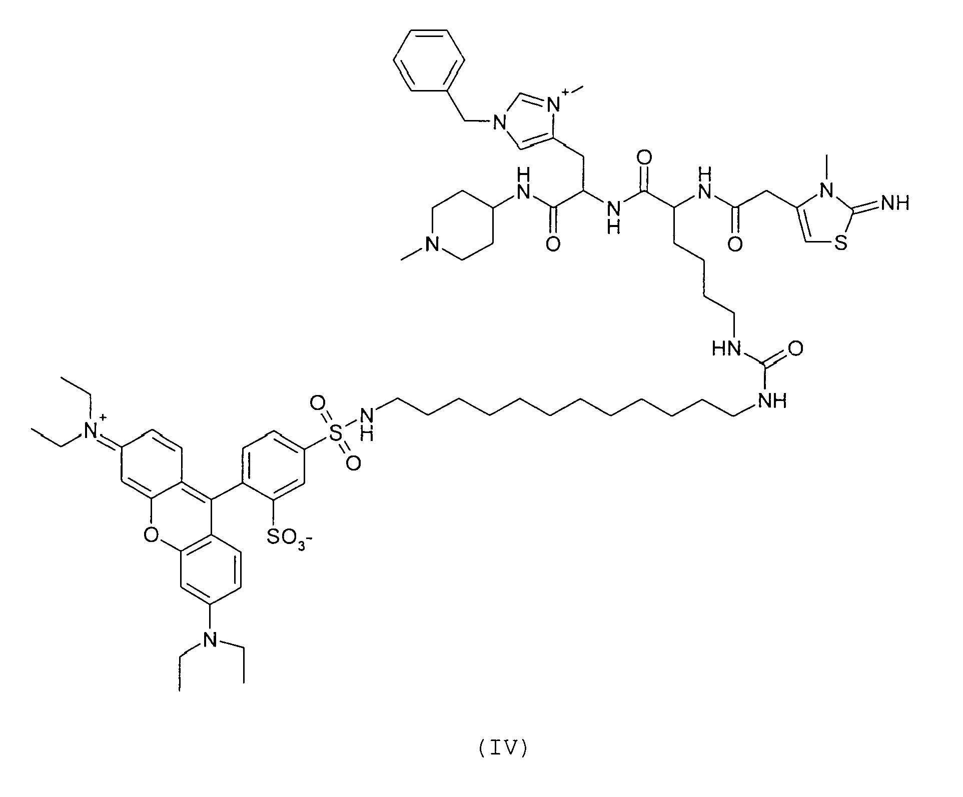

### Synthesis scheme of the compound of formula (IV):

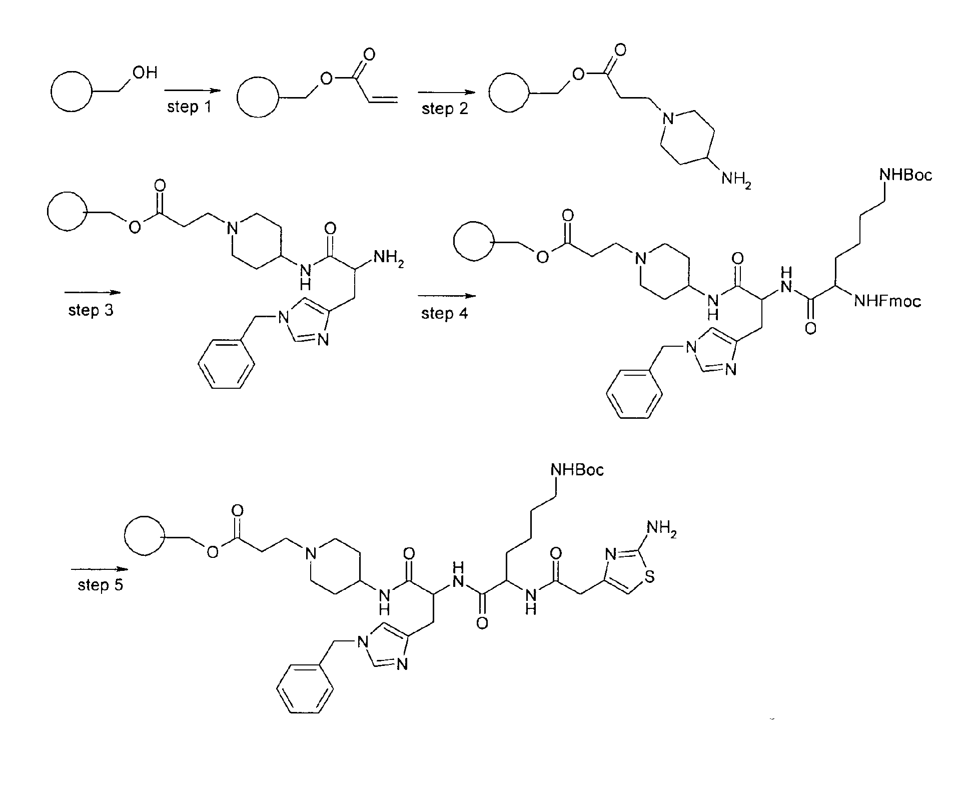

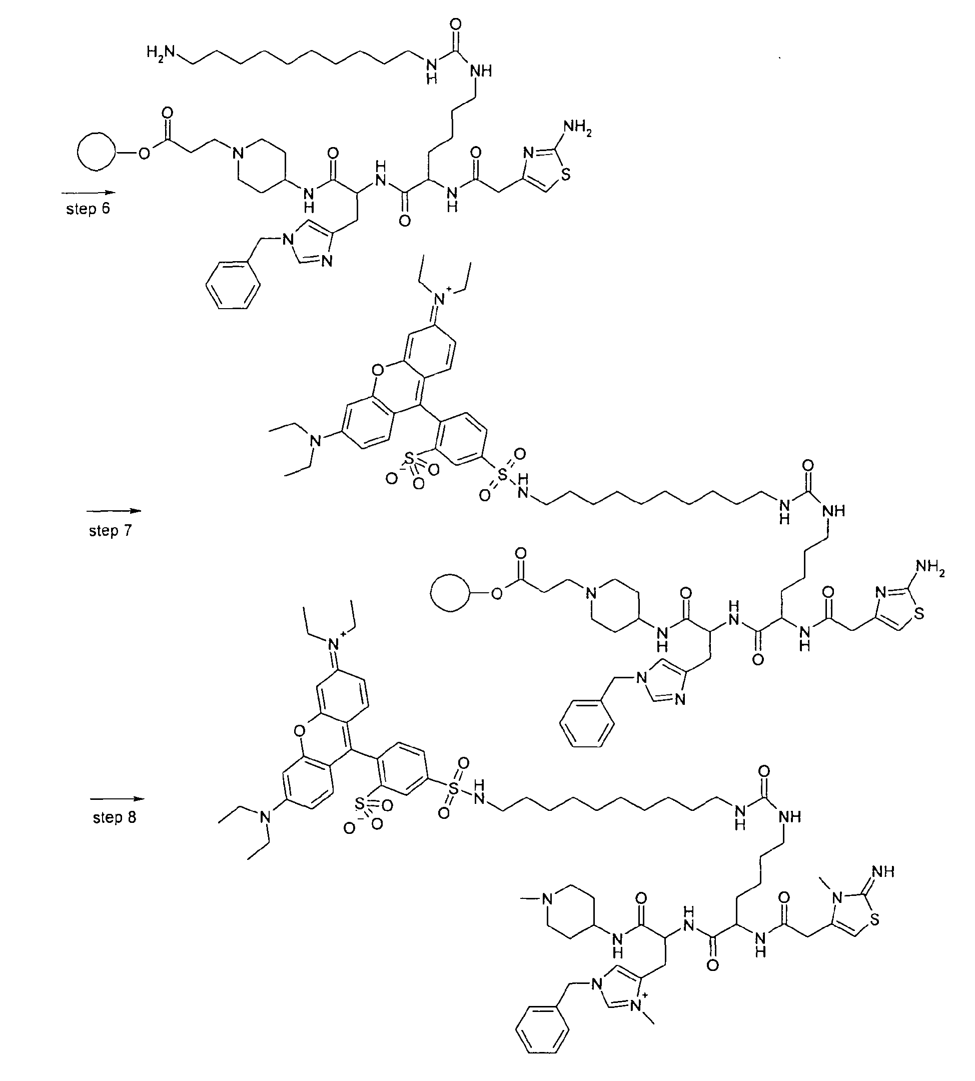

### Step 1: Resin preparation

In 100 ml flask, the resin (1 eq.) was allowed to swell in DCM (10 vol). Diisopropalamine (10 eq.) was added to that suspension. The resulting mixture was shaken at room temperature while acryloyl chloride (10 eq.) was added dropwise.

After shaking for 16 hours at room temperature, the resin was filtered and washed with DMF and DCM. The same procedure was repeated once. The final wash of the resin was carried out with DMF, DCM, MeOH, and Et₂O. The resulting resin was dried under reduced pressure for 16 hours.

The formation of the acrylate was confirmed by IR: C=O 1727, C=C 1403)

### Step 2: Loading of Piperidin-4-ylamine

The resin XX (1 eq.) was allowed to swell in DMF (10 vol.) under N₂, then a solution of diamine (10 eq.) in DMF was added to that suspension. The resulting solution was heated at 80°C for 16 hours. The resin was filtered, washed with DMF, DCM, MeOH and Et₂O. The resulting suspension was allowed to swell in DCM (10 vol), then a solution of TFA/DCM 3:1 (10 vol) was added. After shaking for 1 hour at room temperature, the resin was filtered, washed with DMF, DCM, 10 % DIEA (in DCM), MeOH, and Et₂O. The resin was dried under reduced pressure for 16 hours.

### Step 3: Synthesis of [2-(1-Benzyl-1H-imidazol-4-yl)-1-(1-methyl-piperidin-4-ylcarbamoyl)-ethyl]-carbamic acid tert-butyl ester on resin.

HOBt (3eq.) was added to a solution of acid (3 eq.) in DMF, followed by DIC (3eq). The resulting mixture was stirred for 30 min. In the same time, the resin XX (1eq.) was allowed to swell in DMF (10 eq.) for 30 min. The solution of the activated acid was added to the suspension of resin and was shaken for 6 hours at room temperature. The resin was filtered and washed with DMF, DCM, MeOH and Et₂O. The resulting resin was dried under reduced pressure for 16 hours.

### Cleavage of Boc-protection

The Boc-fonctionnalised resin (1 eq.) was allowed to swell in DCM (3 vol), and then a solution of TFA/DCM 3:1 (7 vol) was added to the suspension. After shaking for 1 hr, the resin was filtered, washed with DCM. The sequence was repeated once. The final wash of the resin was carried out wit DMF, 10 % DIEA (in DCM), MeOH, and Et₂O. The resin was dried under reduced pressure for 16 hours.

### Step 4: Synthesis of 2-Methyl-heptanoic acid [2-(1-benzyl-1H-imidazol-4-yl)-1-(1-methyl-piperidin-4-ylcarbamoyl)-ethyl]-amide on resin

HOBt (3eq.) was added to a solution of *N*-α-Fmoc-*N*-ε-Boc-AA (3 eq.) in DMF, followed by DIC (3eq). The resulting mixture was stirred for 30 min. In the same time, the resin XX (1eq.) was allowed to swell in DMF (10 eq.) for 30 min. The solution of the activated acid was added to the suspension of resin and was allowed to shake for 6hours at room temperature. The resin was filtered and washed with DMF, DCM, MeOH and Et₂O. The resulting resin was dried under reduced pressure for 16 hours.

### Cleavage of Fmoc-protection

The Fmoc-fonctionnalised resin was allowed to swell in DMF (5 vol.) for 15 min, then the excess of DMF was removed by filtration. A piperidin solution (20 % in DMF) was added to the resin and the resulting suspension was shaken for 30 min at room temperature. The resin was filtered and washed using DMF, DCM and DMF. The procedure was repeated once. After 30 min, the resin was filtered and washed using DMF, DCM, MeOH and Et₂O. The resin was finally dried under reduced pressure for 6 hours.

### Step 5: coupling

Using the coupling method described above, (2-Amino-thiazol-4-yl)-acetic acid was coupled to the resin.

### Step 6: formation of the chain

The Boc-fonctionnalised resin (1 eq.) was allowed to swell in DCM (3 vol), and then a solution of TFA/DCM 3:1 (7 vol) was added to the suspension. After shaking for 1 hr, the resin was filtered, washed with DCM. The sequence was repeated once. The final wash of the resin was carried out with DMF, 10 % DIEA (in DCM), MeOH, and Et₂O. The resin was dried under reduced pressure for 16 hours. The extension of the chain was done in two steps. The dry resin (1 eq.) was allowed to swell in DCM for 15 min. A solution of DEA (10 eq.) in DCM/THF (1:1) was added to that suspension, followed by a solution of p-nitrophenylchloroformate in DCM/THF (1:1). The resulting mixture was shaken for 2 hours, the resin was then filtered, washed with DCM and THF. A solution of Diamine (20 eq.) in DCM was added to the resin. After shaking for 15 hours at RT, the resin was filtered washed with DCM, DMF, MeOH and Et₂O. The resin was finally dried under reduced pressure for 6 hours.

### Step 7: coupling to fluorescent group

The resin (1eq.) was allowed to swell in minimum DCM for 15 min. A solution of DIEA (2 eq) in DCM (0.04M) was added to the suspension, followed by a solution of lissamine sulfonyl chloride (2 eq.) in DCM (0.04 M). After shaking 5 hours at RT, the resin was filtered, washed with DCM, DMF, MeOH, and Et₂O. The resin was finally dried under reduced pressure for 6 hours.

### Step 8: Cleavage of the resin

The resin (1 eq.) was allowed to swell in DMF (10 vol) and methyl iodide (30 eq.) was added at room temperature. The resulting suspension was shaken for 20 hours. The resin was filtered, washed with DMF, DCM and DMF. The procedure was repeated once. The resin was filtered, washed with DMF, DCM, MeOH and Et₂O, and dried under reduced pressure. The resin was allowed to swell in DCM (10 vol) and IRA-95 (10 eq.) was added to the suspension. After shaking for 20 hours, the resin was filtered, and washed with MeOH and DCM. The filtrates were collected and evaporated under reduce pressure to afford the desired compound.

The above synthesis scheme, disclosed with respect to the preparation of the compound of formula (III), will also advantageously be used for preparing the compounds of formula (I).

The present invention further provides a pharmaceutical composition comprising a pharmaceutically effective amount of a compound of formula (I), (II), (III) or (IV) or pharmaceutically acceptable salts thereof, in combination with a pharmaceutically acceptable carrier, diluent or excipient.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality.

In another embodiment, the invention provides a pharmaceutical composition for the treatment and/or prophylaxis of a disorders and/or or condition where APJ receptor plays a role.

The compounds of the invention may be administered in a form suitable for oral use, for example a tablet, capsule, aqueous or oily solution, suspension or emulsion; for topical use including transmucosal and transdermal use, for example a cream, ointment, gel, aqueous solution or suspension, salve, patch or plaster; for nasal use for example a snuff, nasal spray or nasal drops; for vaginal or rectal use, for example a suppository; for administration by inhalation, for example a finely divided powder or a liquid aerosol; for sub-lingual or buccal use, for example a tablet or a capsule; or for parenteral use (including intravenous, subcutaneous, intramuscular, intravascular or infusion), for example a sterile aqueous solution or suspension, or depot injection formulation. In general, the above compositions may be prepared in a conventional manner using well known excipients, using standard techniques, including controlled release technologies, such as gelatin, lipid, gel depot, liposome and/or microcapsule based systems well known to those skilled in the art of pharmacy.

For an oral administration, the compounds of the invention will generally be provided in the form of tablets or capsule or as an aqueous solution or suspension.

Tablets for oral use may include the active ingredient mixed with pharmaceutically acceptable excipients such as inert diluents (such as sodium and calcium carbonate, sodium and calcium phosphate, or lactose), disintegrating agents (such as corn starch or alginic acid), binding agents (starch or gelatin), lubricating agents (magnesium stearate, stearic acid or talc), sweetening agents, flavoring agents, coloring agents and preservatives. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or oil such as peanut oil, liquid paraffin or olive oil.

For intramuscular, intraperitoneal, subcutaneous and intravenous use, the compounds of the invention will generally be provided in sterile aqueous solutions (such as Ringer's solution or isotonic sodium chloride) or suspensions (such as cellulose derivatives, sodium alginate, polyvinylpyrrolidone and gum tragacanth with a wetting agent such as lecithin, and a preservative such as ethyl and n-propyl p-hydroxybenzoate), buffered to an appropriate pH and isotonicity.

Transdermal formulations include membrane permeation systems, multi-laminate adhesive dispersion systems and matrix dispersion systems. Transdermal delivery also includes the use of electrically aided transport and skin penetration enhancers.

The preferred route of administration is the intravenous infusion, preferably over a period of up to seven days, or as an oral formulation, or as an intramuscular injection via a styrette or as a subcutaneous injection.

It will be appreciated that the dosage levels used may vary over quite a wide range depending upon the compound used, the severity of the condition exhibited by the patient and the patient's body weight.

In a further embodiment, the invention provides assays for the identification of agents able to modulate APJ ligand binding, signalling and biological activities, where a compound according to the invention is used as a biological tool.

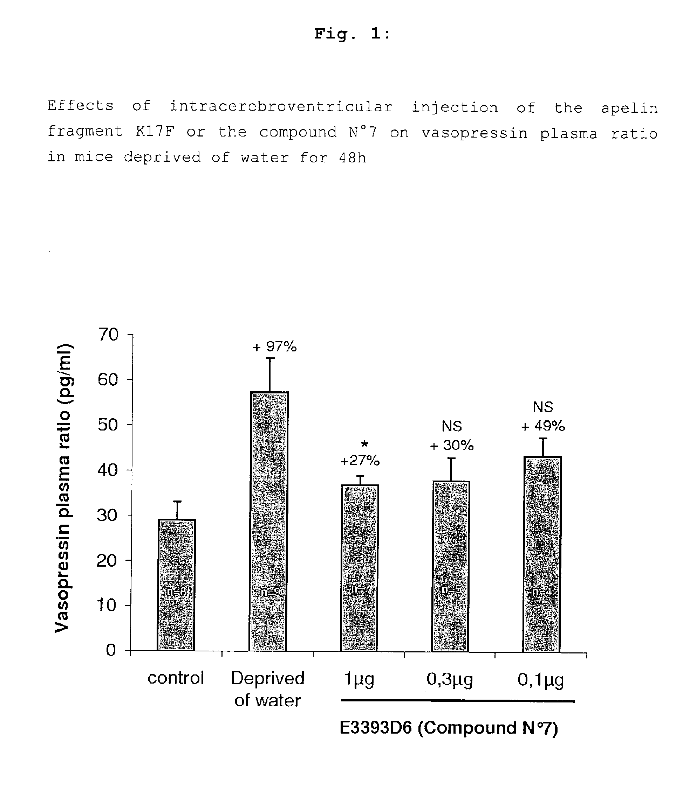

Other characteristics and advantages of the invention are given in the following examples in which reference is made to figures 1 and 2 which represent respectively:
- Figure 1: Effects of intracerebroventricular injection of the apelin fragment K17F or the compound of the invention according to formula (IV) corresponding to compound N°7, on plasma vasopressin levels in mice deprived of water for 48 hours
- Table 1 : Effects of intracerebroventricular injection of the apelin fragment K17F or the compound of the invention according to formula (IV) corresponding to compound code N°7, on plasma vasopressin levels in mice deprived of water for 48 hours

It will be appreciated that the invention is described by way of example only and modification of detail may be made without departing from the scope of the invention.

### Examples:

### Example 1: Inhibition of forskolin-induced cAMP production in CHO cells.

### 1. Test principle

The effect of the compounds of the invention is tested on a CHO cell line which stably expresses the murine APJ receptor. On activation of this cell line with forskolin (FSK) there is an increase in cAMP production leading to an increase in cAMP levels. This increase can be prevented by simultaneous or previous administration of apelin or any agonist ligand to APJ receptor via stimulation of the recombinantly expressed APJ receptor and the inhibition resulting therefrom of adenylate cyclase.

### 2. Test

### CHO cell line expressing the murine APJ receptor

The murine APJ receptor is characterized in the cell line: the APJ receptor is negatively coupled to adenylate cyclase. Fragments of apelin 17 (K17F) and apelin 13 (pE13F) inhibit cAMP production induced by forskolin with an IC₅₀ of 1,3.10⁻¹⁰ M and 2.10⁻¹⁰ M respectively (De Mota et al., Neuroendocrinology, 2000). At concentrations of 10-8 M these fragments are able to induce massive internalisation of the APJ receptor, (Reaux et al., J. Neurochem., 2001; El Messari et al., J. Neurochem., 2004).

### Material and methods

### Measurement of cAMP production in CHO cells stably expressing the rat APJ receptor

CHO cells stably expressing the rat APJ receptor tagged at its C-terminal part with the green fluorescent protein EGFP (APJ-EGFP) are seeded in 24-well plates (100.000 cells/well) the day before the experiment.

The cells are pre-incubated for 2 hours at 37°C in 200 µl of a Krebs-Ringer solution (Hepes 10 mM, NaCl 118 mM, CaCl₂ 2.5 mM, KCl 4.75 mM, MgSO₄ 1.2 mM, KH₂PO₄ 1.2 mM, NaHCO₃ 24 mM, glucose 11.1 mM and BSA 0.1%). The medium is withdrawn and replaced by 200 µl of a Krebs-Ringer solution with IBMX (3-isobutylmethylxanthine, Sigma) at a final concentration of 0.1 mM for an incubation of 20 minutes at 37°C. Forskolin (40 µM) and compounds to be tested are then added (100 µl) at least at four times (4x) the final concentration. Incubation for 30 minutes at 37°C. Reaction is stopped by replacement of the medium by 1.5 ml of cold ethanol (65% v/v, stored at -20°C before use) and storing the plates on ice for 15 minutes. The supernatant (1 ml) is removed and dried under vacuum (Speed-Vac). The pellet is processed by RIA buffer (RIA/cAMP kit, Amersham Biosciences) and stored at +4°C overnight. The cAMP level is determined with a radioimmunoassay (RIA kit, Amersham Biosciences) on diluted samples of 15 to 30 µl. The limit of detection is 25 fmol per tube.

Active fragments of apelin (K17F and pE13F) and compounds of the invention are tested in parallel in order to compare their activities on the APJ receptor.

Various experimental conditions were tested to optimize the technique:
- BSA quantity: adjusted at 0,1% instead of 0,3%,
- Test compounds dilution: in a Krebs-Ringer medium with IBMX (0,1 mM) and DMSO at 6% (Cf=3%),
- Incubation time: 30 minutes at 37°C.

### 3. Results

In the following table, the compound n°3 is the compound of the invention according to formula (III), and the compound n°7 is the compound of the invention according to formula (IV).

| **Compounds** | **Code** | **IC₅₀ (M)** |
|---|---|---|
| 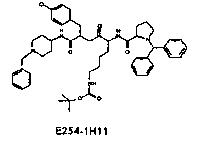 | 1 | 22.8.10⁻⁶ |
| 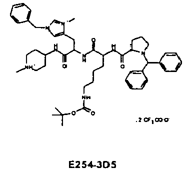 | 2 | 5 ± 0,28.10⁻⁶ |
| 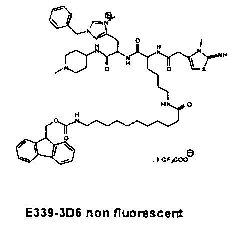 | 3 | 10.10⁻⁶ |
| 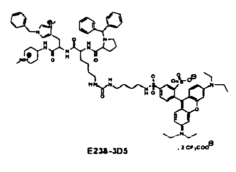 | 4 | 6, 7 ± 0,34.10⁻⁶ |
| 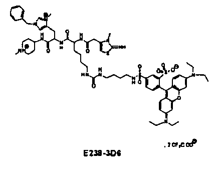 | 5 | 26% à 10.10⁻⁶ |
| 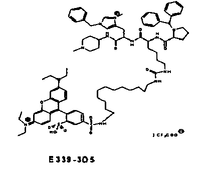 | 6 | 1,1 ± 0,28.10⁻⁶ |
| 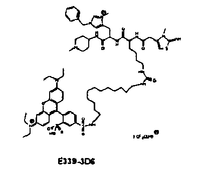 | 7 | 5 ± 0,02. 10⁻⁷ M |
| pE13F (lissamine) | 8 | 5,3 ± 2,1 .10⁻⁸ |
| K17F | 9 | 1,3 ± 0, 4.10⁻¹⁰ |

The compounds 7 and 8 strongly inhibit FSK-induced cAMP production in CHO cells expressing the rat APJ receptor, with an IC50 of 5.10-7 M and 5.10-8 M respectively.

The control compound 9 also inhibits FSK-induced cAMP production in CHO cells with IC50 of 1.3.10-10 M as previously described (Reaux et al. J. Neurochem 2001).

### Example 2:

### 1. Test principle

Internalisation of the APJ receptor in CHO cells.

### 2. Test

The compound 7 as well as the apelin fragments: fluorescent apelin 13 (lissamine) and non fluorescent apelin 13 were tested on the internalization of the murine apelin receptor tagged with EGFP.

### Matérial and methods:

### Internalization assay

Approximately 2.5 x 10⁵ stably transfected CHO cells expressing the rat apelin receptor-EGFP, in Ham's F12 medium, were used to seed polyallylamine hydrochloride-coated (Aldrich Sigma, 0.1 mg/ml for 30 min) 16-well glass slides (Lab-Tek Chamber Slides, Nalge Nunc, Rochester, NY). Cells were cultured overnight in a humidified atmosphere of 5% CO₂ / 95% air. To prevent *de novo* the protein synthesis, cells were incubated for 90 min before plating with 90 µM of cycloheximide at 37°C and cycloheximide was maintained in the medium during and after incubation with ligands. Cells were incubated in ice-cold Earle's buffer, pH 7.4 (140 mM NaCl, 5 mM KCl, 1.8 mM CaCl₂, 3.6 mM MgCl₂) supplemented with 0.2% BSA, 0.1% % glucose, 90 µM cycloheximide and 0.8 mM of 1-10 phenanthroline in the presence of various concentrations of apelin fragments (apelin 17, apelin 13, apelin 10) or the compounds of the invention at 4°C for 30 min. Then, receptor internalization was carried out in Earle's buffer at 37°C for 20 min for the comparison of internalization potency of various compounds. Cells were rinsed three times with 0.1 M phosphate-buffered saline, pH 7.4 (PBS) and then fixed for 10 min at room temperature with 4% % paraformaldehyde dissolved in 0.1 M PBS. They were rinsed again in 0.1M PBS, mounted in Vectashield (Vector Laboratories, Compiègne, France) and covered with a coverslip for confocal microscopy analysis.

### Immunofluorescence and confocal microscopy

Cells were examined with a Leica TCS SP 2 (Leica Microsystems, Heidelberg, Germany) confocal laser scanning microscope mounted on a Leica DM IRBE inverted microscope equipped with an argon/krypton laser. EGFP fluorescence was detected with 100% excitation at 488 nm, using a RSP 500 (dichroic) mirror with the emission wavelength set at 530-560 nm. Dual localization of apelin receptor-EGFP and compound 7, in a given specimen, was acquired sequentially using excitation at 488 nm with the emission wavelength set at 530-560 nm for EGFP, then using excitation at 543 nm and the emission wavelength set at 560-620 nm for lissamine. Optical sections (1024 x 1024) of individual cells were taken at the equatorial level (level of the nucleus), using a 63x 1.32 NA oil-immersion objective.

### Results:

### Endocytosis of the rat apelin receptor

The CHO cell line used in this study was selected for intensity of membrane fluorescence of the apelin receptor-EGFP and K17F-induced inhibition of forskolin-stimulated cAMP production and K17F induced-apelin receptor endocytosis (De Mota et al., Neuroendocrinology, 2000; Reaux et al., J. Neurochem., 2001).

In these cells, both K17F (100 nM) and pE13F (100 nM) inhibit forskolin-stimulated cAMP production induce intense internalization of the rat apelin receptor-EGFP (Reaux et al., J. Neurochem., 2001).

Here, we checked the activities of fluorescent pE13F (lissamine) and non fluorescent pE13F at a concentration of 100nM to induce the internalization of the rat apelin receptor-EGFP. Confocal microscopy showed that after stimulation with 100 nM pE13F-lissamine for 20 min, the complexes pE13F-lissamine-apelin receptor were visualized in numerous fluorescent vesicles distributed through the cytoplasm, (Fig 1). After 20 min of stimulation with 100nM non fluorescent pE13F, the pattern of internalization of the apelin receptor-EGFP was similar to that observed with the CHO cells treated with fluorescent pE13F, indicating that lissamine tagging pE13F did not affect the capacity of pE13F to induce the internalization of the apelin receptor in CHO cells.

### Compound of formula (IV) according to the invention or compound N°7: E339 3D6

The compound 7 at concentrations comprised between 10 and 100 µM induces an internalization of the apelin receptor-EGFP, visualized at the emission wavelength set at 530-560 nm for EGFP (green). This internalization is characterized by a decrease of surface-associated fluorescent label and the appearance of numerous fluorescent intracytoplasmic vesicles but this was not observed in the whole cell population. The internalization observed with the compound 7 is less intense than that observed with pE13F or K17F. Furthermore, another surprising result, not explained is the intracellular labeling visualized at the emission wavelength set at 560-620 nm of lissamine. These data suggest that compound 7 enters in the cells by internalization via the apelin receptor but also via another mechanism which remains to determine.

### Example 3:

### 1. Test principle

The effect of the compound 7 is tested, following its intracerebroventricular (i.c.v.) injection, on the secretion of vasopressin (AVP) in the blood circulation in mice.

### 2. Test

We have previously shown that apelin co-localizes with AVP in SON and PVN magnocellular neurons and given intracerebroventricularly (i.c.v.) acts as a diuretic agent by inhibiting the phasic electrical activity of AVP neurons, thereby decreasing systemic AVP release (De Mota et al., PNAS, 2004)

### Material and methods:

### Intracerebroventricular injections in mice

Drugs dissolved in saline were administered by the i.c.v. route according to the method of Haley and McCormick (Haley and Mc Cormick, Br. J. Pharmacol., 1957). In some experiments, mice were deprived of water for 48 h before use. Alert mice (n=5 animals per group) received a single dose of 10 µl of saline or apelin 17 or the compound 3 (100 ng, 300 ng or 1 µg) or AngII (5 ng). Animals were killed 1 min after the injection, and trunk blood (0.5 - 1 ml) was collected in chilled tubes containing 50 µl of 0.3 M EDTA. Samples were stored on ice for a maximum of 15 min before centrifugation (1600 x g for 15 min) at 4°C. Plasma (0.2 ml) was collected and transferred to polypropylene tubes containing 50 µl of 3 M HCl and was then stored at - 80°C until vasopressin RIA.

### Vasopressin RIA

Vasopressin concentration was determined as previously described (Zini et al., Proc. Natl. Acad. Sci. USA, 1996) from 0.2 ml of plasma, using a specific RIA kit (Peninsula Laboratories, Belmont, CA, USA), using (3-[¹²⁵I]iodotyrosyl-2-vasopressin-[Arg⁸], 2000 Ci / mmol) as the tracer and a polyclonal antiserum specific for vasopressin-[Arg^{B}] with no cross reactivity with oxytocin. Vasopressin was extracted from the plasma with C₁₈ Sep-Pak cartridges (Waters) and measured in duplicate according to the protocol of the kit. The limit of detection of the vasopressin RIA was 0.2 pg per tube.

### Results:

### Effects of intracerebroventricular injection of the apelin fragment K17F or the compound 7 on plasma vasopressin levels in mice deprived of water for 48 hours

Forty-eight hours of water deprivation induced an increase in plasma AVP levels. Under our experimental conditions, plasma AVP levels in water-deprived animals were more than 190% those of mice with free access to water (57.4 ± 7.6, n=9, *versus* 29.1 ± 3.9 pg / ml, n=8) (Fig. 1). In water-deprived mice, i.c.v. injection of the apelin fragment K17F (1µg) alone elicited a significant decrease (-40%) in plasma AVP levels when compared with mice injected with saline (Reaux et al., J. Neurochem., 2001). Similarly, the i.c.v. injection of compound 7 in increasing doses inhibits in a dose-dependent manner plasma AVP levels in water-deprived animals when compared with mice injected with saline (Fig below). The maximal decrease - 38% is obtained for a dose of 1 µg since at a dose of 2 µg, a similar change is observed. To summarize 48h water deprivation results in an increase in plasma vasopressin levels of 97% when compared to mice with free access to water whereas in presence of the compound 7 (1 µg) the increase in plasma vasopressin levels induced by water deprivation is greatly reduced and is only of 27%.

**Table 1: Effects of intracerebroventricular injection of the apelin fragment K17F or the compound 7 on plasma vasopressin levels in mice deprived of water for 48 hours**

| **Mice treatment** | **Vasopressin plasma ratio (pg/ml)** | **Increase compared to control** (%) |
|---|---|---|
| Control | 29,1 ± 4,0 (n=8) | 0 |
| Deprived of water for 48h | 57,4 ± 7,6 (n=9) | 97% |
| Deprived of water for 48h + compound N°7 (1µg) | 36,9 ± 2,0 (n=7) | 27% |
| Deprived of water for 48h + compound N°7 (0,3µg) | 37,9 ± 5,0 (n=5) | 30% |
| Deprived of water for 48h + compound N°7 (0,1µg) | 43,4 ± 4,1 (n=4) | 49% |

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in light of the teachings of this invention that certain changes and modifications may be made thereto without departing from the spirit or scope of the appended claims.

## Claims

1. A compound of formula (I): 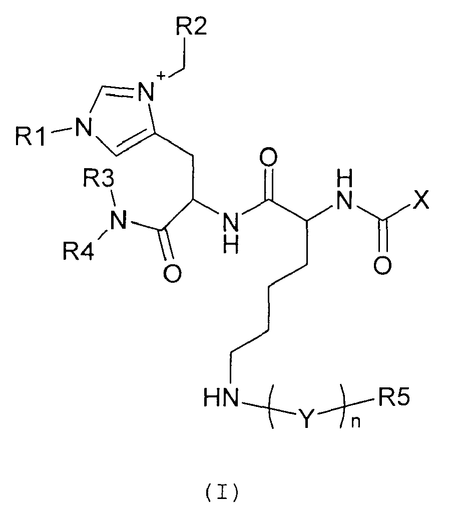 wherein:
**R1** is an aryl, alkylaryl, heteroaryl or alkylheteroaryl group;
**R2** is a hydrogen atom or an aryl group;
**R3** and **R4** represent a hydrogen atom or a heterocycloalkyl group providing that **R3** and **R4** cannot represent simultaneously a hydrogen atom and that **R3** and **R4** can both be part of a heterocycloalkyl group;
**R5** represents a group chosen is the following list: 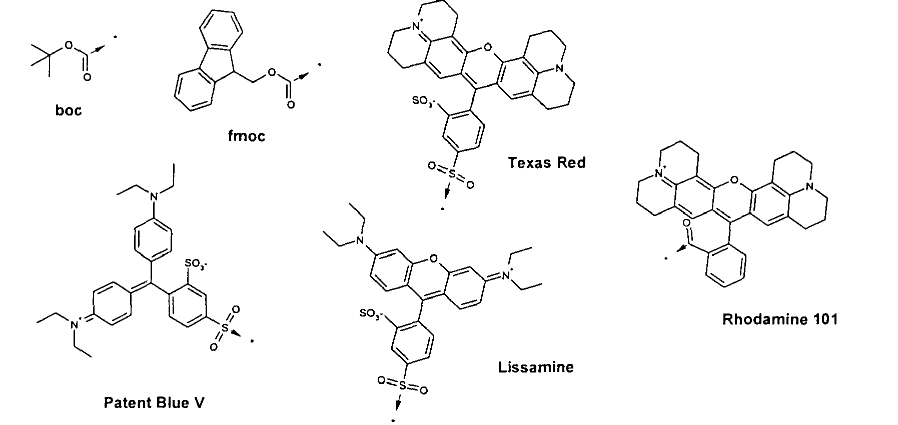
**n** is an integer from 0 to 1
**Y** represents -CO-(NH)_{n'}-A-NH- group with:
**n'** is an integer from 0 to 1;
**A** is a group chosen is the following list:
-(CH₂)_{n"}-
-[(CH₂)₂-O]_{n'"}-(CH₂)₂-
-(CH₂)ₘ-NH-CO-(CH₂)_{m'}-
-(CH₂)ₘ-NH-CO-(CH₂)ₘ,-NH-CO-(CH₂)_{m"}-
-(CH₂)ₘ-CO-NH-(CH₂)_{m'}-
-(CH₂)ₘ-CO-NH-(CH₂)ₘ,-CO-NH-(CH₂)_{m"}-
with n" representing an integer ranging from 1 to 20;
with n"' representing an integer ranging from 1 to 10;
with m, m' and m" representing independently from the other an integer ranging from 1 to 15;
**X** represents a group chosen in the following list: 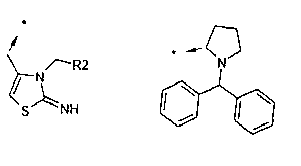 as well as a pharmaceutically acceptable salt thereof, a prodrug of the compound or the salt, or a solvate or hydrate of the compound, the salt or the prodrug.

2. A compound according to claim 1, of formula (II): 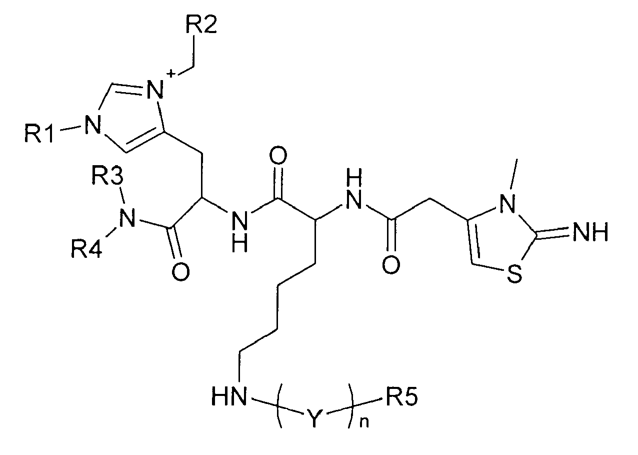 in which:
**R1** to **R5**, **n** and **Y** being as defined in claim 1.

3. A compound according to claim 1 or 2, of formula (III): 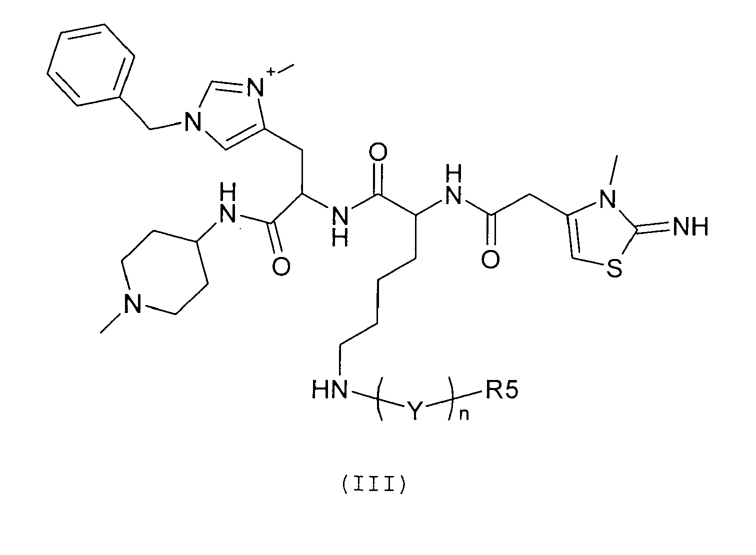 with:
**R5**, **n** and **Y** being as defined in claim 1.

4. A compound according to any one of claims 1 to 3 of formula (IV): 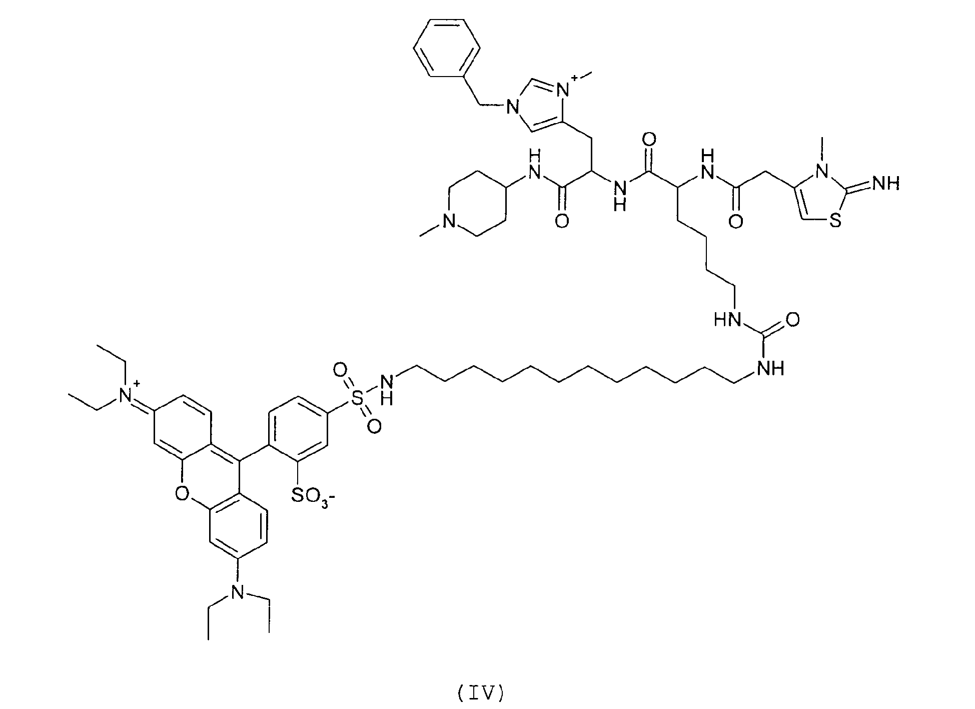 as well as a pharmaceutically acceptable salt thereof, a prodrug of the compound or the salt, or a solvate or hydrate of the compound, the salt or the prodrug.

5. A pharmaceutical composition comprising a pharmaceutically acceptable carrier and a pharmaceutically effective amount of at least one compound of formula (I), (II), (III) or (IV) according to any one of claims 1 to 4.

6. Use of a compound of formula (I), (II), (III) or (IV) according to any one of claims 1 to 4, for the manufacture of a drug for the treatment and/or prophylaxis of diseases and conditions mediated by APJ signalling.
